(19) Europäisches Patentamt
European Patent Office
Office européen des brevets

(11) **EP 4 775 655 A1**

(12) **EUROPEAN PATENT APPLICATION**
published in accordance with Art. 153(4) EPC

(43) Date of publication:
**15.07.2026 Bulletin 2026/29**

(21) Application number: **24877129.7**

(22) Date of filing: **04.10.2024**

(51) International Patent Classification (IPC):
*C10M 105/42* (2006.01)   *C07C 67/08* (2006.01)
*C07C 69/44* (2006.01)   *C10N 30/06* (2006.01)
*C10N 40/30* (2006.01)

(52) Cooperative Patent Classification (CPC):
**C10M 105/42;** C07C 67/08; C07C 69/44;
C10N 2030/06; C10N 2040/30

(86) International application number:
**PCT/JP2024/035673**

(87) International publication number:
**WO 2025/079533 (17.04.2025 Gazette 2025/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.10.2023 JP 2023176827**

(71) Applicant: ENEOS CORPORATION
Chiyoda-ku
Tokyo 100-8162 (JP)

(72) Inventors:
• KAWAGUCHI, Masaki
  Tokyo 100-8162 (JP)
• OGATA, Hidetoshi
  Tokyo 100-8162 (JP)
• SEKI, Yuma
  Tokyo 100-8162 (JP)
• MIZUTANI, Yuya
  Tokyo 100-8162 (JP)

(74) Representative: Grünecker Patent- und
Rechtsanwälte
PartG mbB
Leopoldstraße 4
80802 München (DE)

(54) **COMPLEX ESTER FOR REFRIGERATING MACHINE OILS AND METHOD FOR PRODUCING SAME, REFRIGERATING MACHINE OIL, WORKING FLUID COMPOSITION, AND METHOD FOR IMPROVING STABILITY OF COMPLEX ESTER**

(57)    A complex ester of neopentyl glycol, a dihydric aliphatic alcohol other than neopentyl glycol, a dihydric aliphatic carboxylic acid, and a monohydric aliphatic alcohol, wherein the complex ester has an ester bond formed by esterification of the hydroxy group of the neopentyl glycol and an unreacted hydroxy group in which the hydroxy group has not been esterified, and the proportion of the unreacted hydroxy group in the total of the ester bond and the unreacted hydroxy group is 6% by mole or less, the complex ester being for refrigerating machine oil.

## Description

### Technical Field

The present invention relates to a complex ester for refrigerating machine oil and a method for producing the same, a refrigerating machine oil, a working fluid composition, and a method for improving stability of a complex ester.

### Background Art

Refrigerating machines such as refrigerators, car air conditioners, room air conditioners, and vending machines include a compressor for circulating a refrigerant within a refrigeration cycle. The compressor is filled with a refrigerating machine oil for lubricating sliding members. The refrigerating machine oil contains a base oil and additives. The base oil in the refrigerating machine oil is appropriately selected according to desired properties. As the base oil, for example, an ester obtained by reacting a dihydric alcohol, a dicarboxylic acid, and a monohydric alcohol (a so-called complex ester) may be used.

For example, Patent Literature 1 discloses that an ester obtained from component (A) neopentyl glycol, component (B) a linear dihydric alcohol having hydroxyl groups at both terminal carbons and having 2 to 6 carbon atoms, component (C) a linear dihydric carboxylic acid having carboxyl groups at both terminal carbons and having 4 to 10 carbon atoms, and component (D) a monohydric alcohol having 6 to 12 carbon atoms, in which the number of moles of terminal hydroxyl groups derived from component (A), the number of moles of terminal hydroxyl groups derived from component (B), the number of moles of constituent units derived from component (A), and the number of moles of constituent units derived from component (B) in the ester satisfy specific relationships, has excellent lubricity and heat resistance.

### Citation List

#### Patent Literature

[Patent Literature 1] International Publication No. WO 2016/199718

### Summary of Invention

#### Technical Problem

The present inventors studied ways to improve the stability of complex esters, and found that even by focusing on relationships such as the number of moles of terminal hydroxyl groups derived from component (A) and the number of moles of terminal hydroxyl groups derived from component (B) as disclosed in Patent Literature 1, it is not possible to sufficiently improve the stability of complex esters, particularly the stability when moisture is mixed in.

Accordingly, one aspect of the present invention aims to provide a complex ester for refrigerating machine oil that has excellent stability.

#### Solution to Problem

As a result of their studies, the present inventors found that the ratio of ester bonds formed by esterification of the hydroxy groups of neopentyl glycol (synonymous with hydroxyl groups) to unreacted hydroxy groups in which the hydroxy groups have not been esterified in the complex ester is an important factor that affects the stability of the complex ester. More specifically, it was found that when the proportion of the unreacted hydroxy groups in the total of the ester bonds and the unreacted hydroxy groups in the complex ester is equal to or less than a specific value, the stability of the complex ester is improved compared to when the specific value is exceeded.

The present invention includes the following aspects.

[1] A complex ester for refrigerating machine oil, the complex ester being a complex ester of neopentyl glycol, a dihydric aliphatic alcohol other than neopentyl glycol, a dihydric aliphatic carboxylic acid, and a monohydric aliphatic alcohol, wherein the complex ester has an ester bond formed by esterification of the hydroxy group of the neopentyl glycol and an unreacted hydroxy group in which the hydroxy group has not been esterified, and the proportion of the unreacted hydroxy group in the total of the ester bond and the unreacted hydroxy group is 6% by mole or less.

[2] The complex ester according to [1], wherein the complex ester has an unreacted hydroxy group of the dihydric aliphatic alcohol.

[3] A refrigerating machine oil comprising the complex ester according to [1] or [2].

[4] A working fluid composition comprising the refrigerating machine oil according to [3] and a refrigerant.

[5] A method for improving stability of a complex ester of neopentyl glycol, a dihydric aliphatic alcohol other than neopentyl glycol, a dihydric aliphatic carboxylic acid, and a monohydric aliphatic alcohol, the method comprising: in the complex ester, esterifying the hydroxy group of the neopentyl glycol to generate an ester bond while leaving the hydroxy group unesterified as an unreacted hydroxy group, and setting the proportion of the unreacted hydroxy group in the total of the ester bond and the unreacted hydroxy group to 6% by mole or less to improve the stability.

[6] The method according to [5], wherein an unreacted hydroxy group of the dihydric aliphatic alcohol remains in the complex ester.

[7] A production method of a complex ester, comprising a step of reacting neopentyl glycol, a dihydric aliphatic alcohol other than neopentyl glycol, a dihydric aliphatic carboxylic acid, and a monohydric aliphatic alcohol, wherein in the step, the hydroxy group of the neopentyl glycol is esterified to generate ester bond while leaving the hydroxy group unesterified as an unreacted hydroxy group, and the proportion of the unreacted hydroxy group in the total of the ester bond and the unreacted hydroxy group is set to 6% by mole or less.

[8] The production method according to [7], wherein an unreacted hydroxy group of the dihydric aliphatic alcohol remains in the complex ester.

**Advantageous Effects of Invention**

According to one aspect of the present invention, a complex ester for refrigerating machine oil having excellent stability can be provided. According to another aspect of the present invention, a complex ester for refrigerating machine oil can be provided that has excellent stability, particularly in the presence of a refrigerant and when moisture is mixed in.

**Description of Embodiments**

One embodiment of the present invention is a complex ester of neopentyl glycol, a dihydric aliphatic alcohol other than neopentyl glycol (hereinafter also simply referred to as "dihydric aliphatic alcohol"), a dihydric aliphatic carboxylic acid, and a monohydric aliphatic alcohol.

The dihydric aliphatic alcohol may be linear or branched, and is preferably linear. The number of carbon atoms of the dihydric aliphatic alcohol may be 2 or more or 3 or more, and may be 10 or less, 8 or less, 6 or less, or 4 or less. Examples of such dihydric aliphatic alcohols include ethylene glycol, 1,2-propanediol, 1,3-propanediol, 1,2-butanediol, 1,3-butanediol, 1,4-butanediol, 2,3-butanediol, 1,5-pentanediol, 1,6-hexanediol, 2-methyl-1,3-propanediol, 3-methyl-1,5-pentanediol, and 2,2-diethyl-1,3-pentanediol. The dihydric aliphatic alcohol preferably includes at least one selected from the group consisting of 1,2-butanediol, 1,3-butanediol, 1,4-butanediol, and 2,3-butanediol, more preferably includes at least one selected from the group consisting of 1,3-butanediol and 1,4-butanediol, and still more preferably includes 1,4-butanediol.

The number of carbon atoms of the dihydric aliphatic carboxylic acid may be 5 or more or 6 or more, and may be 12 or less, 10 or less, or 8 or less. Examples of the dihydric aliphatic carboxylic acid include adipic acid, pimelic acid, suberic acid, azelaic acid, and sebacic acid. The dihydric aliphatic carboxylic acid preferably includes at least one selected from adipic acid and sebacic acid, and more preferably includes adipic acid.

The number of carbon atoms of the monohydric aliphatic alcohol may be 4 or more, 6 or more, or 8 or more, and may be 18 or less, 14 or less, or 10 or less. Examples of the monohydric aliphatic alcohol include butanol, pentanol, hexanol, heptanol, octanol, nonanol, decanol, and dodecanol. These monohydric aliphatic alcohols may be linear or branched, and are preferably branched. The monohydric aliphatic alcohol preferably includes at least one selected from the group consisting of octanol and nonanol, more preferably includes at least one selected from the group consisting of branched octanol and branched nonanol, and still more preferably includes at least one selected from the group consisting of 2-ethylhexanol and 3,5,5-trimethylhexanol.

The complex ester is obtained by reacting, per 1 mol of neopentyl glycol, for example, 0.1 to 0.4 mol, preferably 0.1 to 0.3 mol, of the dihydric aliphatic alcohol, 0.8 to 2.8 mol, preferably 1.2 to 2.0 mol, of the dihydric aliphatic carboxylic acid, and 0.3 to 2.3 mol, preferably 0.8 to 1.7 mol, of the monohydric aliphatic alcohol.

The content of units derived from the dihydric aliphatic alcohol per 1 mol of units derived from neopentyl glycol in the complex ester may be 0.1 to 0.4 mol, and may preferably be 0.1 to 0.3 mol. The content of units derived from the dihydric aliphatic carboxylic acid per 1 mol of units derived from neopentyl glycol in the complex ester may be 0.8 to 2.8 mol, and may preferably be 1.2 to 2.0 mol. The content of units derived from the monohydric aliphatic alcohol per 1 mol of units derived from neopentyl glycol in the complex ester may be 0.3 to 2.3 mol, and may preferably be 0.8 to 1.7 mol.

In the complex ester thus obtained, the hydroxy groups of neopentyl glycol, the dihydric aliphatic alcohol, and the monohydric aliphatic alcohol are each esterified by the carboxyl groups of the dihydric aliphatic carboxylic acid to form ester bonds, but a portion of the hydroxy groups of neopentyl glycol remains without being esterified as unreacted hydroxy groups. That is, the complex ester has ester bonds formed by esterification of the hydroxy groups of neopentyl glycol (as well as ester bonds formed by esterification of the respective hydroxy groups of the dihydric aliphatic alcohol and the monohydric aliphatic alcohol) and unreacted hydroxy groups in which the hydroxy groups of neopentyl glycol have not been esterified (hereinafter also referred to as "unreacted hydroxy groups derived from neopentyl glycol").

In this complex ester, the proportion of the unreacted hydroxy groups derived from neopentyl glycol (molar percentage: $X_{OH} = A_{OH}/(A_E + A_{OH}) \times 100$) in the total (the total proportion of which is $A_E + A_{OH}$) of the ester bonds formed by esterification of the hydroxy groups of neopentyl glycol (the proportion of which is $A_E$) and the unreacted hydroxy groups derived from neopentyl glycol (the proportion of which is $A_{OH}$) is 6% by mole or less.

The $X_{OH}$ is preferably 5% by mole or less, 4% by mole or less, 3% by mole or less, or 2% by mole or less from the viewpoint of further improving the stability of the complex ester (particularly the stability in the presence of a refrigerant), and may be 0% by mole, but since complete esterification requires reaction at a higher temperature for a longer period of time, from the standpoint of achieving both economic efficiency and stability, it may be 0.1% by mole or more, 0.2% by mole or more, or 0.3% by mole or more.

The $X_{OH}$ ($= A_{OH}/(A_E + A_{OH}) \times 100$) is determined by $^1$H-NMR measurement. Specifically, from the spectrum obtained by $^1$H-NMR measurement, the proportion of ester bonds formed by esterification of the hydroxy groups of neopentyl glycol ($A_E$) is taken as 1/2 ($I_{AE}$) of the integral value of the peak (around 3.8 to 3.9 ppm) attributed to the hydrogen of the -CH$_2$- (methylene group) adjacent to the ester bonds, and the proportion of unreacted hydroxy groups derived from neopentyl glycol ($A_{OH}$) is taken as 1/2 ($I_{AOH}$) of the integral value of the peak (around 3.0 to 3.5 ppm) attributed to the hydrogen of the -CH$_2$-(methylene group) adjacent to the unreacted hydroxy groups derived from neopentyl glycol, and $I_{AOH}/(I_{AE} + I_{AOH}) \times 100$ calculated using these integral values is defined as the above $X_{OH}$ ($= A_{OH}/(A_E + A_{OH}) \times 100$).

The complex ester may have, in addition to the respective ester bonds described above and the unreacted hydroxy groups derived from neopentyl glycol, unreacted hydroxy groups in which the hydroxy groups of the dihydric aliphatic alcohol have not been esterified (hereinafter also referred to as "unreacted hydroxy groups derived from the dihydric aliphatic alcohol"). In the complex ester according to the present embodiment, even when unreacted hydroxy groups derived from the dihydric aliphatic alcohol remain, the stability of the complex ester (particularly the stability in the presence of a refrigerant) can be improved by virtue of the $X_{OH}$ being 6% by mole or less.

In the complex ester, the proportion of the unreacted hydroxy groups derived from the dihydric aliphatic alcohol (molar fraction: $Y_{OH} = B_{OH}/(A_{OH} + B_{OH})$) in the total (the proportion of which is $A_{OH} + B_{OH}$) of the unreacted hydroxy groups derived from neopentyl glycol (the proportion of which is $A_{OH}$) and the unreacted hydroxy groups derived from the dihydric aliphatic alcohol (the proportion of which is $B_{OH}$) is 0.3 or less, or 0.25 or less, and may be 0, but may be 0.05 or more, 0.10 or more, 0.15 or more, or 0.16 or more.

The $Y_{OH}$ ($= B_{OH}/(A_{OH} + B_{OH})$) is determined by $^1$H-NMR measurement. Specifically, from the spectrum obtained by $^1$H-NMR measurement, the proportion of unreacted hydroxy groups derived from neopentyl glycol ($A_{OH}$) is taken as 1/2 ($I_{AOH}$) of the integral value of the peak (around 3.0 to 3.5 ppm) attributed to the hydrogen of the -CH$_2$-(methylene group) adjacent to the unreacted hydroxy groups derived from neopentyl glycol, and the proportion of unreacted hydroxy groups derived from the dihydric aliphatic alcohol ($B_{OH}$) is taken as 1/2 ($I_{BOH}$) of the integral value of the peak (around 3.6 to 3.8 ppm) attributed to the hydrogen of the -CH$_2$- (methylene group) adjacent to the unreacted hydroxy groups derived from the dihydric aliphatic alcohol, and $I_{BOH}/(I_{AOH} + I_{BOH})$ calculated using these integral values is defined as the above $Y_{OH}$ ($= B_{OH}/(A_{OH} + B_{OH})$).

The complex ester preferably satisfies the following formula:

$$[B_{OH}/(A_{OH} + B_{OH})] / [B_{mol}/(A_{mol} + B_{mol})] > 0.9$$

In the formula, $A_{OH}$ and $B_{OH}$ represent the proportion of unreacted hydroxy groups derived from neopentyl glycol and the proportion of unreacted hydroxy groups derived from the dihydric aliphatic alcohol, respectively, as described above, $A_{mol}$ represents the proportion (molar fraction) of units derived from neopentyl glycol relative to the total amount of units derived from raw materials in the complex ester, and $B_{mol}$ represents the proportion (molar fraction) of units derived from the dihydric aliphatic alcohol relative to the total amount of units derived from raw materials in the complex ester.

The above formula means that the proportion of unreacted hydroxy groups derived from the dihydric aliphatic alcohol in the total of unreacted hydroxy groups derived from neopentyl glycol and unreacted hydroxy groups derived from the dihydric aliphatic alcohol is relatively large relative to the proportion of units derived from the dihydric aliphatic alcohol in the total of the proportion of units derived from neopentyl glycol and the proportion of units derived from the dihydric aliphatic alcohol in the complex ester.

The value of the left-hand side $[B_{OH}/(A_{OH} + B_{OH})] / [B_{mol}/(A_{mol} + B_{mol})]$ of the above formula may preferably be 0.95 or more, 1.0 or more, or 1.1 or more, and may be 2.0 or less, 1.8 or less, or 1.6 or less.

The $[B_{mol}/(A_{mol} + B_{mol})]$ in the above formula is determined by $^{13}$C-NMR measurement. When the complex ester of the present invention uses 1,4-butanediol as the dihydric aliphatic alcohol, specifically, from the spectrum obtained by

$^{13}$C-NMR measurement, the proportion of units derived from neopentyl glycol is taken as 1/2 ($I_{Amol}$) of the integral value of the peak (around 21.0 to 22.0 ppm) attributed to the carbons of the two -CH$_3$ (methyl groups) derived from neopentyl glycol, and the proportion of units derived from the dihydric aliphatic alcohol is taken as 1/4 ($I_{Bmol}$) of the integral value of the peak (around 25.0 to 26.0 ppm and around 63.0 to 64.0 ppm) attributed to the carbons of the four -CH$_2$- (methylene groups) derived from the dihydric aliphatic alcohol (1,4-butanediol), and $I_{Bmol}/(I_{Amol} + I_{Bmol})$ calculated using these values is defined as the above ($B_{mol}/(A_{mol} + B_{mol})$).

The kinematic viscosity of the complex ester at 40°C may be 10 mm$^2$/s or more, 30 mm$^2$/s or more, or 50 mm$^2$/s or more, and may be 1000 mm$^2$/s or less, 500 mm$^2$/s or less, 300 mm$^2$/s or less, or 200 mm$^2$/s or less. The kinematic viscosity of the complex ester at 100°C may be 1 mm$^2$/s or more or 2 mm$^2$/s or more, and may be 100 mm$^2$/s or less or 50 mm$^2$/s or less. The viscosity index of the complex ester may be 100 or more, 120 or more, or 130 or more, and may be 200 or less or 160 or less. The kinematic viscosity and viscosity index in the present specification mean the kinematic viscosity and viscosity index measured in accordance with JIS K2283:2000.

The acid value of the complex ester may be 1 mgKOH/g or less, 0.1 mgKOH/g or less, 0.05 mgKOH/g or less, or 0.02 mgKOH/g or less. The acid value in the present specification means the acid value measured in accordance with JIS K2501:2003.

In the complex ester as described above, the stability (particularly the stability in the presence of a refrigerant) is improved by the $X_{OH}$ (= $A_{OH}/(A_E + A_{OH}) \times 100$) being 6% by mole or less. That is, another embodiment of the present invention is a method for improving the stability of a complex ester of neopentyl glycol, a dihydric aliphatic alcohol other than neopentyl glycol, a dihydric aliphatic carboxylic acid, and a monohydric aliphatic alcohol, the method including: in the complex ester, esterifying the hydroxy groups of neopentyl glycol to generate ester bonds while leaving the hydroxy groups of the neopentyl glycol unesterified as unreacted hydroxy groups, and improving the stability by setting the proportion of the unreacted hydroxy groups in the total of the ester bonds and the unreacted hydroxy groups to 6% by mole or less.

Such a complex ester is obtained by a method for producing a complex ester that includes a step of reacting neopentyl glycol, a dihydric aliphatic alcohol other than neopentyl glycol, a dihydric aliphatic carboxylic acid, and a monohydric aliphatic alcohol. In the step, the hydroxy groups of neopentyl glycol are esterified to generate ester bonds while leaving the hydroxy groups of neopentyl glycol unesterified as unreacted hydroxy groups, and the proportion of the unreacted hydroxy groups in the total of the ester bonds and the unreacted hydroxy groups is set to 6% by mole or less.

More specifically, neopentyl glycol, a dihydric aliphatic alcohol other than neopentyl glycol, a dihydric aliphatic carboxylic acid, and a monohydric aliphatic alcohol are charged into a suitable reactor, and an esterification reaction is carried out at normal pressure under a nitrogen atmosphere in the presence of an acid catalyst (a Brønsted acid catalyst, a Lewis acid catalyst, or the like) or without a catalyst. The esterification reaction is carried out at, for example, 130°C to 260°C, preferably 150 to 250°C, in order to efficiently remove the water produced by the reaction. Since neopentyl glycol has a melting point of about 130°C and the reaction for esterifying both terminal ends does not proceed readily at 150°C or below, it is preferable to carry out the reaction at 150°C or above, 160°C or above, 180°C or above, or 200°C or above. Note that the monohydric aliphatic alcohol may be added to the reaction system in a slight excess after the neopentyl glycol-dihydric aliphatic carboxylic acid ester units have grown, to esterify the terminal carboxylic acid groups and sufficiently reduce the acid value. By doing so, it is possible to increase the neopentyl glycol-dihydric aliphatic carboxylic acid ester units, reduce the unreacted terminal hydroxy groups, and efficiently increase the kinematic viscosity of the complex ester. On the other hand, when the reaction temperature exceeds 260°C, thermal decomposition reactions tend to occur and efficiency deteriorates, so it is preferable to carry out the reaction at 250°C or below or 240°C or below. The reaction time depends on the reaction temperature, but is, for example, 1 to 24 hours, preferably 2 to 10 hours, and is adjusted so that the kinematic viscosity and the proportion of unreacted hydroxy groups in the units derived from neopentyl glycol are equal to or less than predetermined values. The resulting complex ester is subjected to distillation under reduced pressure to remove excess moisture and unreacted components, and is purified by treating with an adsorbent or the like to adsorb the catalyst and unstable components, and the adsorbent is then removed by decantation or filtration.

The complex ester described above is suitably used in refrigerating machine oil (complex ester for refrigerating machine oil), and is particularly suitably used as a base oil for refrigerating machine oil. That is, another embodiment of the present invention may be a refrigerating machine oil containing the above complex ester, or may be a base oil for refrigerating machine oil containing the above complex ester.

The complex ester may be used as a sole base oil or may be used in partial blending (in combination with other base oils). The content of the complex ester in the refrigerating machine oil may be 100% by mass (sole base oil) based on the total amount of the base oil for refrigerating machine oil, and in the case of partial blending, may be 5% by mass or more, 10% by mass or more, 20% by mass or more, 30% by mass or more, 40% by mass or more, 50% by mass or more, 60% by mass or more, 70% by mass or more, 80% by mass or more, or 90% by mass or more, and may be 99 % by mass or less, 90% by mass or less, 80% by mass or less, 70% by mass or less, 60% by mass or less, 50% by mass or less, 40% by mass or less, or 30% by mass or less.

The content of the complex ester may be 5% by mass or more, 10% by mass or more, 20% by mass or more, 30% by mass or more, 40% by mass or more, 50% by mass or more, 60% by mass or more, 70% by mass or more, 80% by mass or more, or 90% by mass or more based on the total amount of the refrigerating machine oil, and may be 99% by mass or less, 90% by mass or less, 80% by mass or less, 70% by mass or less, 60% by mass or less, 50% by mass or less, 40% by mass or less, or 30% by mass or less.

The refrigerating machine oil may further contain other base oils other than the above complex ester. Examples of other base oils include known base oils such as oxygen-containing oils other than the above complex ester and hydrocarbon oils. Examples of oxygen-containing oils include esters and ethers other than the above complex ester. Examples of esters other than the above complex ester include polyol esters, esters of a monohydric alcohol with a monobasic acid or a polybasic acid, and complex esters other than the above complex ester (for example, complex esters using trimethylolpropane, pentaerythritol, or the like as a polyol other than neopentyl glycol). Examples of ethers include polyalkylene glycols and polyvinyl ethers. Examples of hydrocarbon oils include mineral oils, alkylbenzenes, alkylnaphthalenes, poly-α-olefins, polybutenes, and ethylene-α-olefin copolymers.

The refrigerating machine oil preferably further contains, in addition to the above complex ester, a polyol ester other than the complex ester. Examples of the polyol ester include esters of a polyhydric alcohol and a fatty acid.

The polyhydric alcohol may be a polyhydric alcohol having 2 to 6 hydroxy groups. The number of carbon atoms of the polyhydric alcohol may be 4 or more or 5 or more, and may be 12 or less or 10 or less. Examples of the polyhydric alcohol include neopentyl glycol, trimethylolethane, trimethylolpropane, trimethylolbutane, di(trimethylolpropane), tri(trimethylolpropane), pentaerythritol, and dipentaerythritol, and the like. The polyhydric alcohol preferably includes pentaerythritol, and may include pentaerythritol and dipentaerythritol.

The fatty acid may be linear or branched. The fatty acid may be a saturated fatty acid or an unsaturated fatty acid. The number of carbon atoms of the fatty acid may be 4 or more or 5 or more, and may be 20 or less, 18 or less, or 9 or less. Examples of the fatty acid include 2-methylpropanoic acid, 2-methylbutanoic acid, 3-methylbutanoic acid, 2-methylpentanoic acid, 2-methylhexanoic acid, 2-ethylpentanoic acid, 2-methylheptanoic acid, 2-ethylhexanoic acid, 3,5,5-trimethylhexanoic acid, oleic acid, stearic acid, and 2-ethylhexadecanoic acid, and the like. The fatty acid preferably includes at least one selected from the group consisting of 2-methylpropanoic acid, 2-ethylhexanoic acid, and 3,5,5-trimethylhexanoic acid.

The content of the polyol ester may be 5% by mass or more, 10% by mass or more, 20% by mass or more, 30% by mass or more, 40% by mass or more, 50% by mass or more, 60% by mass or more, 70% by mass or more, 80% by mass or more, or 90% by mass or more based on the total amount of the base oil for refrigerating machine oil, and may be 99% by mass or less, 90% by mass or less, 80% by mass or less, 70% by mass or less, 60% by mass or less, 50% by mass or less, 40% by mass or less, or 30% by mass or less.

The content of the polyol ester may be 5% by mass or more, 10% by mass or more, 20% by mass or more, 30% by mass or more, 40% by mass or more, 50% by mass or more, 60% by mass or more, 70% by mass or more, 80% by mass or more, or 90% by mass or more based on the total amount of the refrigerating machine oil, and may be 99% by mass or less, 90% by mass or less, 80% by mass or less, 70% by mass or less, 60% by mass or less, 50% by mass or less, 40% by mass or less, or 30% by mass or less.

The refrigerating machine oil may further contain additives. Examples of the additives include antiwear agents, antioxidants, acid scavengers, oiliness agents, metal deactivators, viscosity index improvers, pour point depressants, and detergent dispersants. The total content of these additives may be, for example, 0.5% by mass or more, 1% by mass or more, or 2% by mass or more based on the total amount of the refrigerating machine oil, and may be 40% by mass or less, 25% by mass or less, 10% by mass or less, 5% by mass or less, 4% by mass or less, or 3% by mass or less.

The refrigerating machine oil may exist in a refrigerating machine as a working fluid composition for a refrigerating machine, in the form of a mixture with a refrigerant. That is, in one embodiment, the refrigerating machine oil is used together with a refrigerant. Another embodiment of the present invention is a working fluid composition containing the above refrigerating machine oil and a refrigerant.

Examples of the refrigerant include saturated fluorinated hydrocarbons (also referred to as HFC), unsaturated fluorinated hydrocarbons (also referred to as HFO), hydrocarbons, fluorine-containing ethers, bis(trifluoromethyl) sulfide, trifluoroiodomethane, ammonia, and carbon dioxide. In one embodiment, the refrigerant includes a saturated fluorinated hydrocarbon. In one embodiment, the refrigerant includes an unsaturated fluorinated hydrocarbon. In one embodiment, the refrigerant includes a saturated fluorinated hydrocarbon and an unsaturated fluorinated hydrocarbon.

The saturated fluorinated hydrocarbon is preferably a saturated fluorinated hydrocarbon having 1 to 3 carbon atoms, more preferably 1 to 2 carbon atoms. Examples of the saturated fluorinated hydrocarbon include difluoromethane (R32), trifluoromethane (R23), pentafluoroethane (R125), 1,1,2,2-tetrafluoroethane (R134), 1,1,1,2-tetrafluoroethane (R134a), 1,1,1-trifluoroethane (R143a), 1,1-difluoroethane (R152a), fluoroethane (R161), 1,1,1,2,3,3,3-heptafluoropropane (R227ea), 1,1,1,2,3,3-hexafluoropropane (R236ea), 1,1,1,3,3,3-hexafluoropropane (R236fa), 1,1,1,3,3-pentafluoropropane (R245fa), and 1,1,1,3,3-pentafluorobutane (R365mfc). The saturated fluorinated hydrocarbon preferably includes difluoromethane (R32).

The unsaturated fluorinated hydrocarbon is preferably an unsaturated fluorinated hydrocarbon refrigerant having 2 to 4 carbon atoms, and examples thereof include fluoropropene refrigerants having 3 carbon atoms and 1 to 5 fluorine atoms such as 2,3,3,3-tetrafluoropropene (R1234yf), 1,2,3,3,3-pentafluoropropene (R1225ye), 1,3,3,3-tetrafluoropropene (R1234ze), 1,2,3,3-tetrafluoropropene (R1234ye), and 3,3,3-trifluoropropene (HFO-1243zf), fluoroethylene refrigerants having 2 carbon atoms and 1 to 3 fluorine atoms such as monofluoroethylene (HFO-1141), 1,1-difluoroethylene (HFO-1132a), (E)-1,2-difluoroethylene (HFO-1132(E)), (Z)-1,2-difluoroethylene (HFO-1132(Z)), and 1,1,2-trifluoroethylene (R1123), fluorobutene refrigerants having 4 carbon atoms and 1 to 7 fluorine atoms such as (E)-1,1,1,4,4,4-hexafluoro-2-butene (R1336mzz(E)) and (Z)-1,1,1,4,4,4-hexafluoro-2-butene (R1336mzz(Z)), and unsaturated fluorinated hydrocarbon refrigerants containing chlorine atoms and fluorine atoms such as 1-chloro-2,2-difluoroethylene (HCFO-1122), (Z)-1-chloro-2,3,3,3-tetrafluoropropene (HCFO-1224yd(Z)), and (E)-1-chloro-2,3,3,3-tetrafluoropropene (HCFO-1224yd(E)).

The hydrocarbon is preferably a hydrocarbon having 1 to 5 carbon atoms, more preferably a hydrocarbon having 2 to 4 carbon atoms. Examples of the hydrocarbon include methane, ethylene, ethane, propylene, propane (R290), cyclopropane, normal butane, isobutane, cyclobutane, methylcyclopropane, 2-methylbutane, and normal pentane. The hydrocarbon is preferably at least one selected from the group consisting of propane, normal butane, isobutane, and 2-methylbutane.

The refrigerant may be a mixed refrigerant of two or more selected from the above saturated fluorinated hydrocarbon refrigerants, unsaturated fluorinated hydrocarbon refrigerants, and natural refrigerants, and examples of saturated fluorinated hydrocarbon mixed refrigerants include R404A, R407C, R407E, R410A, R410B, and R507C, and examples of unsaturated fluorinated hydrocarbon mixed refrigerants include, as mixed refrigerants containing the above unsaturated fluorinated hydrocarbons having 2 to 4 carbon atoms, R444A, R444B, R445A, R446A, R447A, R447B, R448A, R448B, R449A, R449B, R449C, R450A, R451A, R451B, R452A, R452B, R452C, R454A, R454B, R454C, R454D, R455A, R455B, R455C, R456A, R457A, R457B, R457C, R457D, R459A, R459B, R460A, R460B, R460C, R463A, R463A-J, R464A, R465A, R468A, R468B, R468C, R470A, R470B, R471A, R473A, R474A, R474B, R475A, R476A, R477B, R479A, R480A, R481A, R482A, R486A, R488A, R491A, R513A, R513B, R514A, R515A, R515B, R516A, and other mixed refrigerants containing unsaturated fluorinated hydrocarbons having 2 to 4 carbon atoms. The GWP of such unsaturated fluorinated hydrocarbon refrigerants or unsaturated fluorinated hydrocarbon mixed refrigerants may be, for example, 1500 or less, 1000 or less, 500 or less, 300 or less, 150 or less, 100 or less, or 10 or less.

The content of the refrigerating machine oil in the working fluid composition for a refrigerating machine may be 1 part by mass or more or 2 parts by mass or more, and may be 500 parts by mass or less or 400 parts by mass or less, per 100 parts by mass of the refrigerant.

The refrigerating machine oil and the working fluid composition for a refrigerating machine are suitably used in refrigerating machines having reciprocating or rotary hermetic compressors, such as air conditioners, refrigerators, open-type or closed-type car air conditioners, dehumidifiers, water heaters, freezers, refrigerated and frozen warehouses, vending machines, showcases, and chemical plants, and in refrigerating machines having centrifugal compressors.

**Examples**

Hereinafter, the present invention will be described more specifically based on examples, but the present invention is not limited to the examples.

(Example 1)

As raw materials, 104 g of neopentyl glycol, 15 g of 1,4-butanediol, and 262 g of adipic acid were charged into a four-necked flask, and an ester intermediate was obtained by reacting at normal pressure under a nitrogen stream at 220°C while distilling off the water of reaction, to which 227 g of 3,5,5-trimethylhexanol was further added and reacted, and the remaining unreacted components and moisture were distilled off under reduced pressure. The resulting ester was cooled, subjected to adsorption treatment with activated clay and a silica-alumina-based adsorbent, and filtered using a 1 $\mu$m filter to obtain the target complex ester (kinematic viscosity at 40°C: 131.7 mm$^2$/s, kinematic viscosity at 100°C: 17.8 mm$^2$/s, viscosity index 150, acid value: less than 0.01 mgKOH/g).

In addition, as a result of $^1$H-NMR and $^{13}$C-NMR analysis, the units derived from neopentyl glycol were 24% by mole, the units derived from 1,4-butanediol were 4% by mole (0.17 mol per 1 mol of units derived from neopentyl glycol), the units derived from adipic acid were 43 % by mole (1.79 mol per 1 mol of units derived from neopentyl glycol), and the units derived from 3,5,5-trimethylhexanol were 29% by mole (1.21 mol per 1 mol of units derived from neopentyl glycol). The value of $X_{OH}$ (= $A_{OH}/(A_E + A_{OH}) \times 100$) was 1.5% by mole, the value of $Y_{OH}$ (= $B_{OH}/(A_{OH} + B_{OH})$) was 0.15, the value of $B_{mol}/(A_{mol} + B_{mol})$ was 0.14, and the value of $[B_{OH}/(A_{OH} + B_{OH})] / [B_{mol}/(A_{mol} + B_{mol})]$ was 1.07.

(Comparative Example 1)

A complex ester (kinematic viscosity at 40°C: 137.9 mm$^2$/s, kinematic viscosity at 100°C: 17.7 mm$^2$/s, viscosity

index 142, acid value: less than 0.01 mgKOH/g) was obtained by the same procedure as in Example 1, except that 104 g of neopentyl glycol, 13 g of 1,4-butanediol, 225 g of adipic acid, and 168 g of 3,5,5-trimethylhexanol were charged into a four-necked flask, and the reaction was carried out at normal pressure under a nitrogen stream while distilling off the water of reaction in two stages, first at 120°C and then at 220°C.

In addition, as a result of $^1$H-NMR and $^{13}$C-NMR analysis, the units derived from neopentyl glycol were 28% by mole, the units derived from 1,4-butanediol were 4% by mole, the units derived from adipic acid were 43% by mole, and the units derived from 3,5,5-trimethylhexanol were 25% by mole. The value of $X_{OH}$ (= $A_{OH}/(A_E + A_{OH}) \times 100$) was 7.7% by mole, the value of $Y_{OH}$ (= $B_{OH}/(A_{OH} + B_{OH})$) was 0.12, the value of $B_{mol}/(A_{mol} + B_{mol})$ was 0.13, and the value of $[B_{OH}/(A_{OH} + B_{OH})] / [B_{mol}/(A_{mol} + B_{mol})]$ was 0.89.

(Example 2)

A complex ester (kinematic viscosity at 40°C: 149.2 mm$^2$/s, kinematic viscosity at 100°C: 19.5 mm$^2$/s, viscosity index 150, acid value: less than 0.01 mgKOH/g) was obtained by the same procedure as in Example 1, except that the raw materials were changed to use 104 g of neopentyl glycol, 14 g of 1,4-butanediol, and 242 g of adipic acid, and 227 g of 3,5,5-trimethylhexanol was changed to 175 g of 2-ethylhexanol. In addition, as a result of $^1$H-NMR and $^{13}$C-NMR analysis, the units derived from neopentyl glycol were 26% by mole, the units derived from 1,4-butanediol were 4% by mole (0.15 mol per 1 mol of units derived from neopentyl glycol), the units derived from adipic acid were 43% by mole (1.65 mol per 1 mol of units derived from neopentyl glycol), and the units derived from 2-ethylhexanol were 27% by mole (1.04 mol per 1 mol of units derived from neopentyl glycol). The value of $X_{OH}$ (= $A_{OH}/(A_E + A_{OH}) \times 100$) was 0.7% by mole, the value of $Y_{OH}$ (= $B_{OH}/(A_{OH} + B_{OH})$) was 0.21, the value of $B_{mol}/(A_{mol} + B_{mol})$ was 0.13, and the value of $[B_{OH}/(A_{OH} + B_{OH})] / [B_{mol}/(A_{mol} + B_{mol})]$ was 1.59.

(Comparative Example 2)

A complex ester (kinematic viscosity at 40°C: 155.4 mm$^2$/s, kinematic viscosity at 100°C: 19.7 mm$^2$/s, viscosity index 146, acid value: less than 0.01 mgKOH/g) was obtained by the same procedure as in Comparative Example 1, except that the raw materials were changed to use 104 g of neopentyl glycol, 12.4 g of 1,4-butanediol, and 212 g of adipic acid, and 168 g of 3,5,5-trimethylhexanol was changed to 140 g of 2-ethylhexanol. In addition, as a result of $^1$H-NMR and $^{13}$C-NMR analysis, the units derived from neopentyl glycol were 29% by mole, the units derived from 1,4-butanediol were 4% by mole, the units derived from adipic acid were 42% by mole, and the units derived from 2-ethylhexanol were 24% by mole. The value of $X_{OH}$ (= $A_{OH}/(A_E + A_{OH}) \times 100$) was 7.3% by mole, the value of $Y_{OH}$ (= $B_{OH}/(A_{OH} + B_{OH})$) was 0.08, the value of $B_{mol}/(A_{mol} + B_{mol})$ was 0.12, and the value of $[B_{OH}/(A_{OH} + B_{OH})] / [B_{mol}/(A_{mol} + B_{mol})]$ was 0.68.

(Stability Evaluation 1)

Stability Evaluation 1 was carried out in accordance with JIS K2211-09 (autoclave test). Specifically, 80 g of a sample (each of the complex esters of the examples and comparative examples with the moisture content adjusted to 500 ppm) was weighed into an autoclave, a catalyst (iron, copper, and aluminum wires, each with an outer diameter of 1.6 mm × length of 50 mm) and 20 g of a refrigerant (R32) were sealed therein, and the mixture was heated at 175°C for 168 hours. The acid value (JIS C2101) of the sample after heating was measured. The results are shown in Table 1.

[Table 1]

|  | Example 1 | Comparative Example 1 | Example 2 | Comparative Example 2 |
|---|---|---|---|---|
| $X_{OH}$ (=$A_{OH}/(A_E+A_{OH})\times100$) (mol%) | 1.5 | 7.7 | 0.7 | 7.3 |
| Stability Evaluation 1 Acid value (mgKOH/g) | 0.24 | 1.36 | 0.08 | 0.80 |

(Stability Evaluation 2)

For Examples 1 and 2 and Comparative Example 2, Stability Evaluation 2 was carried out in the same manner as Stability Evaluation 1, except that the refrigerant in Stability Evaluation 1 was changed from R32 to R454B (R32/R1234yf = 69/31 (mass ratio), GWP = 464). The acid values after heating were 0.96 mgKOH/g for Example 1, 0.65 mgKOH/g for Example 2, and 1.23 mgKOH/g for Comparative Example 2.
As described above, the complex ester for refrigerating machine oil described above exhibits excellent stability (particularly stability in the presence of a refrigerant containing a saturated fluorinated hydrocarbon refrigerant and/or an unsaturated fluorinated hydrocarbon refrigerant) even as a standalone complex ester, by virtue of the value of $X_{OH}$ (= $A_{OH}/(A_E + A_{OH}) \times 100$) being 6% by mole or less, as compared to a case where the value exceeds 6% by mole.

It was confirmed that even when the units derived from 1,4-butanediol per 1 mol of units derived from neopentyl glycol in the complex ester of Example 1 were changed to 0.1 mol or 0.3 mol, the units derived from adipic acid were changed to 1.2 mol or 2.0 mol, and the units derived from 3,5,5-trimethylhexanol were changed to 0.8 mol or 1.7 mol, the complex ester exhibits excellent stability (particularly stability in the presence of a refrigerant containing a saturated fluorinated hydrocarbon refrigerant and/or an unsaturated fluorinated hydrocarbon refrigerant) even as a standalone complex ester, because the value of $X_{OH}$ (= $A_{OH}/(A_E + A_{OH}) \times 100$) is 6% by mole or less, compared to a case where the value exceeds 6% by mole.

It was confirmed that even when the combination of neopentyl glycol/1,4-butanediol/adipic acid/3,5,5-trimethylhexanol in the complex ester of Example 1 was changed to the following combinations:

- neopentyl glycol/1,2-butanediol/adipic acid/2-ethylhexanol
- neopentyl glycol/1,3-butanediol/sebacic acid/2-ethylhexanol
- neopentyl glycol/1,2-butanediol/adipic acid/3,5,5-trimethylhexanol
- neopentyl glycol/1,4-butanediol/adipic acid/normal heptanol the complex ester exhibits excellent stability (particularly stability in the presence of a refrigerant containing a saturated fluorinated hydrocarbon refrigerant and/or an unsaturated fluorinated hydrocarbon refrigerant) even as a standalone complex ester, by virtue of the value of $X_{OH}$ (= $A_{OH}/(A_E + A_{OH}) \times 100$) being 6% by mole or less, compared to when the value exceeds 6% by mole.

(Example 3)

As a base oil, in addition to 10% by mass of the complex ester of Example 2, 90% by mass of a polyol ester-based base oil (a base oil containing, as a main component, a tetraester of pentaerythritol with 2-methylpropanoic acid and 3,5,5-trimethylhexanoic acid) was used to prepare refrigerating machine oil base oil 1 (kinematic viscosity at 40°C: 64 mm$^2$/s, kinematic viscosity at 100°C: 8.4 mm$^2$/s, acid value: 0.01 mKOH/g or less). To this refrigerating machine oil base oil 1, additives including a glycidyl ester-based acid scavenger (glycidyl neodecanoate), a phenolic antioxidant (DBPC), and a phosphorus-based antiwear agent (TCP) were blended in a total amount of 2% by mass based on the total amount of the refrigerating machine oil to prepare refrigerating machine oil 1 (kinematic viscosity at 40°C: 63 mm$^2$/s, kinematic viscosity at 100°C: 8.3 mm$^2$/s, acid value: 0.01 mKOH/g or less).

As a result of carrying out Stability Evaluation 1 and Stability Evaluation 2 described above on the obtained refrigerating machine oil 1, the acid value after heating was 0.01 mgKOH/g or less in both Stability Evaluation 1 and Stability Evaluation 2.

In addition, as a result of carrying out Stability Evaluation 3 on the refrigerating machine oil 1 in the same manner as Stability Evaluation 1, except that the refrigerant in Stability Evaluation 1 was changed from R32 to R410A (R32/R125 = 50/50 (mass ratio)), the acid value after heating was 0.01 mgKOH/g or less.

Note that the two-layer separation temperature (TOP) of the mixture of the refrigerating machine oil 1 and the refrigerant was +10°C or less even in the case of R32, and was -30°C or less in both the cases of R410A and R454B. That is, the complex ester for refrigerating machine oil described above exhibits good stability and miscibility (particularly stability and miscibility in the presence of a refrigerant containing a saturated fluorinated hydrocarbon refrigerant and/or an unsaturated fluorinated hydrocarbon refrigerant) even in a refrigerating machine oil blended with a polyol ester and additives. Therefore, in one embodiment, the complex ester (and furthermore, a refrigerating machine oil in which a polyol ester and additives are blended with the complex ester) is also useful as a shared oil for saturated fluorinated hydrocarbon refrigerants and/or unsaturated fluorinated hydrocarbon refrigerants.

**Claims**

1. A complex ester for refrigerating machine oil, the complex ester being a complex ester of neopentyl glycol, a dihydric aliphatic alcohol other than neopentyl glycol, a dihydric aliphatic carboxylic acid, and a monohydric aliphatic alcohol, wherein the complex ester has an ester bond formed by esterification of the hydroxy group of the neopentyl glycol and an unreacted hydroxy group in which the hydroxy group has not been esterified, and the proportion of the unreacted hydroxy group in the total of the ester bond and the unreacted hydroxy group is 6% by mole or less.

2. The complex ester according to claim 1, wherein the complex ester has an unreacted hydroxy group of the dihydric aliphatic alcohol.

3. A refrigerating machine oil comprising the complex ester according to claim 1 or 2.

4. A working fluid composition comprising the refrigerating machine oil according to claim 3 and a refrigerant.

5. A method for improving stability of a complex ester of neopentyl glycol, a dihydric aliphatic alcohol other than neopentyl glycol, a dihydric aliphatic carboxylic acid, and a monohydric aliphatic alcohol, the method comprising: in the complex ester, esterifying the hydroxy group of the neopentyl glycol to generate an ester bond while leaving the hydroxy group unesterified as an unreacted hydroxy group, and setting the proportion of the unreacted hydroxy group in the total of the ester bond and the unreacted hydroxy group to 6% by mole or less to improve the stability.

6. The method according to claim 5, wherein an unreacted hydroxy group of the dihydric aliphatic alcohol remains in the complex ester.

7. A production method of a complex ester, comprising a step of reacting neopentyl glycol, a dihydric aliphatic alcohol other than neopentyl glycol, a dihydric aliphatic carboxylic acid, and a monohydric aliphatic alcohol, wherein in the step, the hydroxy group of the neopentyl glycol is esterified to generate an ester bond while leaving the hydroxy group unesterified as an unreacted hydroxy group, and the proportion of the unreacted hydroxy group in the total of the ester bond and the unreacted hydroxy group is set to 6% by mole or less.

8. The production method according to claim 7, wherein an unreacted hydroxy group of the dihydric aliphatic alcohol remains in the complex ester.

## INTERNATIONAL SEARCH REPORT

| International application No. |
| --- |
| **PCT/JP2024/035673** |

| A. | CLASSIFICATION OF SUBJECT MATTER |
| --- | --- |

*C10M 105/42*(2006.01)i; *C07C 67/08*(2006.01)n; *C07C 69/44*(2006.01)n; *C10N 30/06*(2006.01)n; *C10N 40/30*(2006.01)n
FI: C10M105/42; C07C67/08; C07C69/44; C10N40:30; C10N30:06

According to International Patent Classification (IPC) or to both national classification and IPC

| B. | FIELDS SEARCHED |
| --- | --- |

Minimum documentation searched (classification system followed by classification symbols)

C10M101/00-177/00; C07B31/00-63/04; C07C1/00-409/44

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2016/199718 A1 (NOF CORPORATION) 15 December 2016 (2016-12-15) paragraphs [0062], [0067], [0069], tables 2, 4, comparative example 2 | 1-4, 7-8 |
| A | | 5-6 |
| A | WO 2021/005986 A1 (JXTG NIPPON OIL & ENERGY CORPORATION) 14 January 2021 (2021-01-14) claims 1-4 | 1-8 |
| A | WO 2016/002523 A1 (JX NIPPON OIL & ENERGY CORPORATION) 07 January 2016 (2016-01-07) claims 1-8 | 1-8 |
| A | WO 2013/129566 A1 (JX NIPPON OIL & ENERGY CORPORATION) 06 September 2013 (2013-09-06) claims 1-12 | 1-8 |
| A | JP 2001-507334 A (EXXON CHEMICAL PATENTS INC.) 05 June 2001 (2001-06-05) claims 1-25 | 1-8 |

☐ Further documents are listed in the continuation of Box C.    ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **22 November 2024** | **03 December 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/035673**

| Patent document cited in search report | | | Publication date (day/month/year) | Patent family member(s) | | | Publication date (day/month/year) |
|---|---|---|---|---|---|---|---|
| WO | 2016/199718 | A1 | 15 December 2016 | EP 3305878 A1 paragraph [0067], tables 2, 4, comparative example 2 | | | |
| | | | | CN | 107614663 | A | |
| | | | | KR | 10-2018-0017079 | A | |
| | | | | TW | 201700721 | A | |
| WO | 2021/005986 | A1 | 14 January 2021 | US 2022/0325159 A1 claims 1-4 | | | |
| | | | | CN | 114072487 | A | |
| WO | 2016/002523 | A1 | 07 January 2016 | JP | 2020-158786 | A | |
| | | | | TW | 201606070 | A | |
| WO | 2013/129566 | A1 | 06 September 2013 | US 2015/0014575 A1 claims 1-24 | | | |
| | | | | JP | 2013-181133 | A | |
| | | | | EP | 2821464 | A1 | |
| | | | | CN | 104145006 | A | |
| | | | | KR | 10-2014-0133828 | A | |
| | | | | TW | 201343898 | A | |
| JP | 2001-507334 | A | 05 June 2001 | WO 1998/010040 A1 claims 1-25 | | | |
| | | | | US | 5750750 | A | |
| | | | | EP | 925339 | A1 | |
| | | | | AU | 4183797 | A | |
| | | | | BR | 9712807 | A | |
| | | | | CA | 2262466 | A1 | |
| | | | | CN | 1229425 | A | |
| | | | | KR | 10-2001-0029458 | A | |
| | | | | AR | 9726 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016199718 A